Europäisches Patentamt

European Patent Office

Office européen des brevets

(11) Publication number: **0 092 982**
**A2**

# EUROPEAN PATENT APPLICATION

(21) Application number: **83302277.5**

(22) Date of filing: **21.04.83**

(51) Int. Cl.³: **A 61 B 5/04**
**A 61 B 5/00**

(30) Priority: **23.04.82 US 371372**

(43) Date of publication of application:
**02.11.83 Bulletin 83/44**

(84) Designated Contracting States:
**AT BE CH DE FR GB IT LI LU NL SE**

(71) Applicant: **AMERICAN HOME PRODUCTS CORPORATION**
**685, Third Avenue**
**New York, New York 10017(US)**

(72) Inventor: **Hon, Edward Harry Gee**
**11, Bradbury Hills Road**
**Bradbury California 91010(US)**

(74) Representative: **Chettle, Adrian John et al,**
**c/o John Wyeth & Brother Limited Huntercombe Lane South**
**Taplow Maidenhead Berkshire, SL6 0PH(GB)**

(54) Detection of fetal death.

(57) Systems and methods are provided for diagnosing the death of a fetus in utero. A first ECG signal from the fetus is detected. A second ECG signal from the mother simultaneously is detected. The two ECG signals are displayed on a common time base and compared to determine whether an ECG component originating with the fetus is present in the first ECG signal.

FIG.1

Croydon Printing Company Ltd.

EP 0 092 982 A2

# DETECTION OF FETAL DEATH

The present invention relates to systems and methods for diagnosing the death of a fetus in utero.

Due to the limitations of existing monitoring systems, the diagnosis of fetal death in utero is frequently very difficult. For example, it is not always possible to obtain a fetal heart motion signal ultrasonically despite the fact that the fetus is alive. A more reliable method for obtaining a fetal heart signal is the direct monitoring of fetal ECG detected by a fetal scalp electrode of the type subject of U.S. Patent Re. 28,990 to Edward H. Hon et al. However, the direct monitoring of fetal ECG is complicated by the electrically conductive path between the mother's circulatory system and that of the fetus through the placenta. Consequently, the maternal ECG can be detected by the fetal scalp electrode and mistaken for the fetal ECG when displayed by the fetal monitor.

The obstetrician may conclude from the observation of the maternal ECG on the fetal monitor that the fetus is alive, although he has confused the maternal ECG for that of the fetus. This is of particular importance when the observed heart rate is slow. The obstetrician may conclude that the fetus is in distress as indicated by the low heart rate. Unfortunately, this may not be the case and a cesarian section is performed to save the life of the fetus which, in fact, is already dead. Had the obstetrician known that the fetus actually was dead, he could have permitted the labor to progress to a vaginal delivery and avoided the risks and trauma to the mother of a cesarian delivery.

In accordance with one aspect of the present invention, a system is provided for diagnosing the death of a fetus in utero. The system comprises first means for receiving an ECG signal from the fetus; second means for receiving an ECG signal from the mother; characterized in that the system further comprises means for displaying the ECG signal from the fetus and the ECG signal from the mother on a common time base. Accordingly, the obstetrician is thus able to compare the ECG signal from the fetus which may be obtained, for example, via a fetal scalp electrode, with the ECG signal which is obtained directly from the mother to reliably determine whether the two signals are the same (in which case the fetus is likely to be dead), or whether the signals are in fact distinct (in which case the fetus is likely still to be alive).

In accordance with a further aspect of the present invention, a method is provided of diagnosing the death of a fetus in utero. The method comprises the steps of: detecting a first ECG signal from the fetus; simultaneously detecting a second ECG signal from the mother; characterized in that the method further comprises the steps of displaying both ECG signals on a common time base; and comparing the ECG signals to determine whether an ECG component originating with the fetus is present in the first ECG signal.

The present invention, as well as further objects and features thereof, will be understood more clearly and fully from the following description of certain preferred embodiments when read with reference to the companying drawings, in which:

Figure 1 is a partially cut-away view of components for use in the system of the present invention, the components being in position for detecting the ECG signals both from a fetal presenting part and the maternal cervical wall, both of which being shown in cross-section, and of a leg plate anchored to a maternal leg (shown partially cut-away), the leg plate providing a means for positioning the components and terminating electrical leads therefrom; and

Figure 2 is a schematic diagram of a system for diagnosing the death of a fetus in utero.

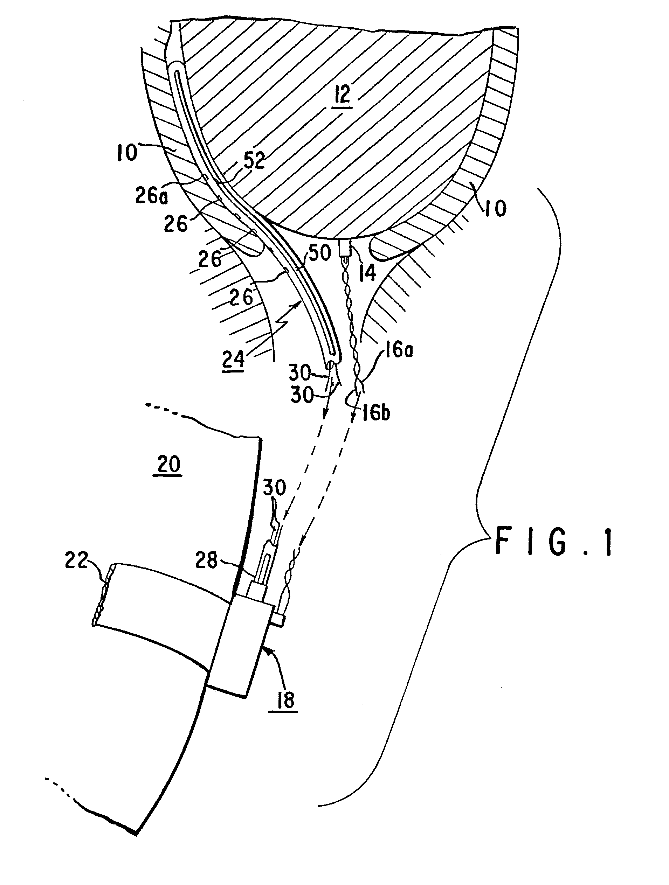

With reference first to Figure 1, a partially dilated cervix of a woman in labor is shown in cross section as 10 and a fetal presenting part is shown also in cross section as 12, the membranes having already been broken. A fetal scalp electrode holder 14 of the type disclosed in U.S. Reissue Patent No. 28,990 is shown affixed to the fetal presenting part 12 for detecting the fetal ECG signal.

Leads 16a and 16b from electrode holder 14 serve to couple its spiral electrode (embedded in the fetal presenting part 12) and its reference electrode, respectively, to terminals provided on a leg plate 18 anchored to a maternal leg 20 by a circumferential strap 22.

Also illustrated in Figure 1 is a generally elongated device 24 for monitoring cervical dilatation during labor and positioned between the cervix 10 and the fetal presenting part 12 for this purpose. The device 24 includes a plurality of ECG electrodes 26 spaced at equal intervals along the longitudinal axis of the device 24 and on its outer surface for contacting the wall of the cervix 10. The device 24 has a proximal end 28 anchored to leg plate 18 to prevent the device 24 from moving longitudinally with respect to the anchored leg plate 18. Each of electrodes 26 has a respective wire 30 connected thereto and leading toward the proximal end 28 at which it is connected to a respective terminal of leg plate 18. Those of the electrodes 26 which are in contact with the wall of the cervix 10 will detect the maternal ECG signal therefrom. Accordingly, by monitoring the signals from the individual electrodes 26, it is possible to monitor the dilatation of the cervix 10 as labor progresses. The maternal ECG signal may be detected with the use of any of the devices disclosed in our U.S. Patent Application No. 371371 entitled "Monitoring of Cervical Dilation During Labor" and filed on 23rd April, 1982.which are provided with cervical ECG electrodes.

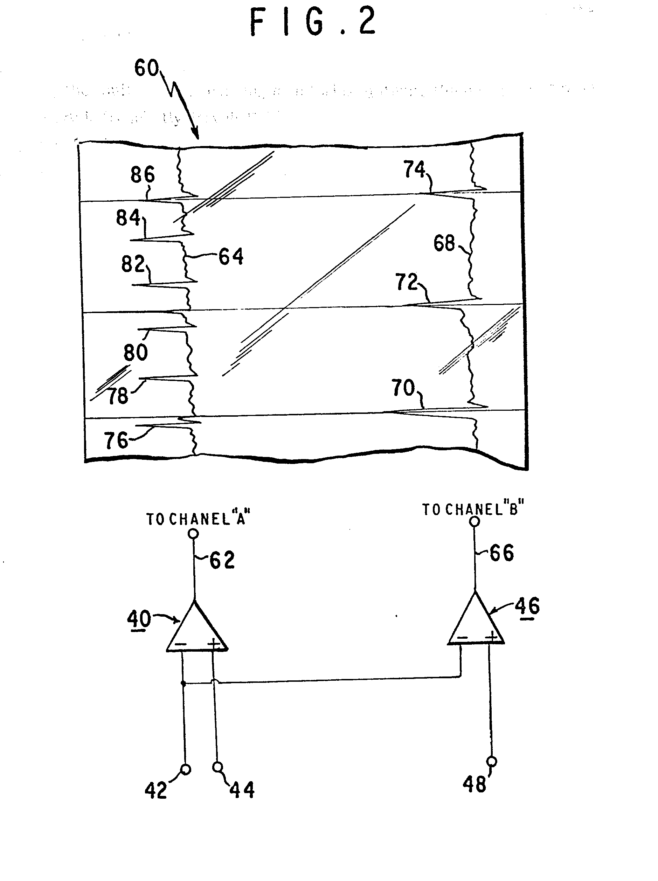

With reference now to Figure 2, a first differential amplifier 40 has a inverting input coupled with an input terminal 42 and a non-inverting input coupled with an input terminal 44. Terminal 42 is coupled to the terminal of leg plate 18 which is connected to lead 16b and, thus, to the reference electrode of holder 14. Terminal 44 is coupled to the terminal of leg plate 18 connected to the lead 16a and, thus, to the spiral electrode of holder 14. Accordingly, amplifier 40 is coupled to receive the ECG signal from the fetus.

A second differential amplifier 46 has an inverting input coupled to input terminal 42 and, thus, to the reference electrode of holder 14. Amplifier 46 has a non-inverting input coupled with an input terminal 48 which in turn is coupled with one of electrodes 26 through a respective terminal of leg plate 18 and wire 30. Said one electrode 26 preferably is that electrode, indicated as 26a, which is closest to the distal end of device 24. Accordingly, amplifier 26 is coupled to receive the ECG signal from the mother.

The ECG signal from the mother may also be detected by other means. In accordance with an advantageous embodiment of the present invention, an intrauterine pressure catheter 50, adapted to sense intrauterine pressure in the lower uterine segment is shown affixed in side-by-side relationship with the device 24 (refer to Figure 1). An ECG electrode 52 is positioned on an outer surface of catheter 50 and disposed to contact the wall of the cervix 10 to detect the maternal ECG signal therefrom. In this embodiment, electrode 52 is coupled to terminal 48 to provide the maternal ECG signal thereto. The side-by-side positioning of device 24 and catheter 50 aids in maintaining the electrodes 26 and 52 in position to contact the wall of the cervix 10.

A strip chart recorder having two channels, A and B, for recording two respective signals on a common time base, is represented as a partially cut away portion of a strip chart record 60. Amplifier 40 has an output terminal 62 to which the ECG signal from the fetal scalp electrode is provided. Terminal 62 is coupled to channel A of the strip chart recorder and is displayed thereby as a tracing 64. Amplifier 46 has an output terminal 66 to which the ECG signal from one of electrodes 26 and 52 is provided. Terminal 66 is coupled to channel B of the strip chart recorder and is displayed thereby as a tracing 68.

Since the tracings 64 and 68 are recorded on a common time base, the obstetrician is enabled to compare simultaneous portions of the two tracings to determine whether the fetal scalp electrode is providing an ECG signal originating with the fetus or originating with the mother. For example, by comparing the tracings 64 and 68 of Figure 2, the obstetrician would be informed in this particular case that R-waves 70, 72 and 74 of tracing 68 are clearly distinct from R waves 76 through 86 of tracing 64, which must therefore be of fetal origin. It is most likely, therefore, that the fetus was alive at the time the tracings 64 and 68 were obtained. Had the tracings 64 and 68 been identical, however, it is likely that at that time the fetus no longer was alive.

It will be appreciated that various other modes of displaying the ECG signals may be employed. For example, they may be displayed on a two channel, non-fade oscilloscope. It will also be appreciated that the maternal ECG signal may be obtained from various other maternal locations and by various other means.

The terms and expressions which have been employed are used as terms of description and not of limitation, and there is no intention in the use of such terms and expressions of excluding any equivalents of the features shown and described, or portions thereof, it being recognized that various modifications are possible within the scope of the invention claimed.

I claim:

1. A system for diagnosing the death of a fetus in utero, comprising: first means for receiving an ECG signal from the fetus; second means for receiving an ECG signal from the mother; characterized in that the system further comprises means for displaying the ECG signal from the fetus and the ECG signal from the mother on a common time base.

2. The system of claim 1, further comprising an electrode adapted to contact a maternal cervix to detect an ECG signal therefrom and means for coupling the detected ECG signal to the second means.

3. The system of claim 2, wherein the electrode is mounted on a device adapted to be positioned between a fetal presenting part and the cervix.

4. The system of claim 3, wherein the device is an intrauterine pressure catheter.

5. The system of claim 5, wherein the device is adapted for monitoring cervical dilatation.

6. A method of diagnosing the death of a fetus in utero, comprising the steps of: detecting a first ECG signal from the fetus; simultaneously detecting a second ECG signal from the mother; characterized in that the method further comprises the steps of: displaying both ECG signals on a common time base; and comparing the ECG signals to determine whether an ECG component originating with the fetus is present in the first ECG signal.

7. The method of claim 6, wherein the second ECG signal is detected from the mother's cervix.

F I G . 1

# FIG.2